# FASCICULE DE BREVET EUROPEEN

(11) **EP 1 030 648 B1**
(45) Date de publication et mention de la délivrance du brevet: **03.12.2003**
(21) Numéro de dépôt: 99923653.2
(22) Date de dépôt: 02.06.1999
(51) Int. Cl.: A61K 7/13

(54) **COMPOSITION TINCTORIALE CONTENANT UNE PYRAZOLO- 1,5-a]-PYRIMIDINE A TITRE DE BASE D'OXYDATION ET UN COUPLEUR PYRIDINIQUE, ET PROCEDES DE TEINTURE**
FÄRBEMITTEL ENTHALTEND PYRAZOLO(1,5-A)PYRIMIDINDERIVATE ALS OXIDATIONSMITTEL UND PYRIDINDERIVATE ALS KUPPLER SOWIE FÄRBEVERFAHREN
DYEING COMPOSITION CONTAINING A PYRAZOLO- 1,5-a]-PYRIMIDINE AS OXIDATION BASE AND A PYRIDINE COUPLING AGENT, AND DYEING METHOD

(30) Priorité: 19.06.1998 FR 9807797
(43) Date de publication de la demande: 30.08.2000
(73) Titulaire: L'OREAL, 75008 Paris (FR)
(72) Inventeur: AUDOUSSET, Marie-Pascale, F-92600 Asnières (FR)
(74) Mandataire: Goulard, Sophie
(86) Numéro de dépôt international: FR9901294
(87) Numéro de publication internationale: WO99066894

(56) Documents cités:
- WO-A-97/35550
- WO-A-97/49378
- WO-A-98/51268
- WO-A-98/55083
- DE-A- 4 029 324
- DE-A- 4 133 957

## Description

L'invention a pour objet une composition pour la teinture d'oxydation des fibres kératiniques contenant au moins une pyrazolo-[1,5-a]-pyrimidine à titre de base d'oxydation et au moins un coupleur pyridinique; ainsi que le procédé de teinture d'oxydation mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bis-phénylalkylènediamines ou bien encore des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénois et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, en particulier dans la demande de brevet FR-A-2 750 048, d'utiliser des pyrazolo-[1,5-a]-pyrimidines à titre de base d'oxydation, seules ou en association avec un ou plusieurs coupleurs. Cependant, les colorations obtenues ne sont pas toujours assez puissantes, chromatiques ou résistantes aux différentes agressions que peuvent subir les cheveux.

Or, la Demanderesse vient maintenant de découvrir, de façon totalement inattendue et surprenante, que l'association des pyrazolo-[1,5-a]-pyrimidines de formule (I) ci-après définie et d'au moins un coupleur pyridinique de formule (II) ci-après définie permettait d'obtenir des colorations puissantes présentant de plus des propriétés de résistance améliorées vis à vis des diverses agressions que peuvent subir les cheveux (shampooings, lumière, intempéries, ondulations permanentes, transpiration, frottements, etc...).

Ces découvertes sont à la base de la présente invention.

L'invention a donc pour premier objet une composition pour la teinture d'oxydation des fibres kératiniques, et en particulier des fibres kératiniques humaines telles que les cheveux, caractérisée par le fait qu'elle contient, dans un milieu approprié pour la teinture :
- au moins une base d'oxydation choisie parmi les pyrazolo-[1,5-a]-pyrimidines de formule (I) suivante et leurs sels d'addition avec un acide ou avec une base : 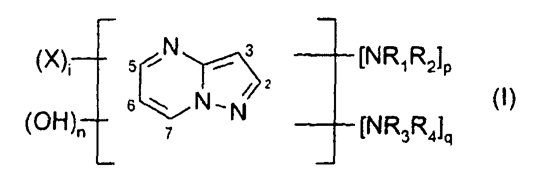
dans laquelle ;
- R₁, R₂ R₃ et R₄ désignent , identiques ou différents un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical amino alkyle en C₁-C₄ (l'amine pouvant être protégée par un acétyle, un uréido, un sulfonyl), un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C₄)alkyl- ou di-[hydroxy(C₁-C₄) alkyl]-amino alkyle en C₁-C₄ ;
- les radicaux X, identiques ou différents, désignent un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C₄)alkyl ou di-[hydroxy(C₁-C₄)alkyl]amino alkyle en C₁-C₄, un radical amino, un radical (C₁-C₄)alkyl- ou di-[(C₁-C₄)alkyl]-amino ; un atome d'halogène, un groupe acide carboxylique, un groupe acide sulfonique ;

- i vaut 0, 1, 2 ou 3 ;
- p vaut 0 ou 1 ;
- q vaut 0 ou 1 ;
- n vaut 0 ou 1 ;
sous réserve que :
- (i) la somme p + q est différente de 0 ;
- (ii) lorsque p + q est égal à 2, alors n vaut 0 et les groupes NR₁R₂ et NR₃R₄ occupent les positions (2,3) ; (5,6) ; (6,7) ; (3,5) ou (3,7) ;
- (iii) lorsque p + q est égal à 1 alors n vaut 1 et le groupe NR₁R₂ (ou NR₃R₄) et le groupe OH occupent les positions (2,3) ; (5,6) ; (6,7) ; (3,5) ou (3,7) ;

- et au moins un coupleur choisi parmi les pyridines de formule (II) suivante, et leurs sels d'addition avec un acide : 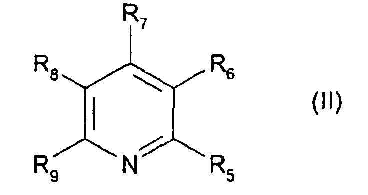
dans laquelle :
- R₅ représente un atome d'hydrogène, un radical hydroxyle, amino, alcoxy en C₁-C₄, mono- ou di-alky(C₁-C₄)amino, monohydroxyalkyl(C₁-C₄)amino, polyhydroxyalkyl(C₂-C₄)amino, monohydroxyalcoxy en C₁-C₄, polyhydroxyalcoxy en C₂-C₄, alcoxy en C₁-C₄, ou monohydroxyalcoxy(C₁-C₄)alcoxy en C₁-C₄ ;
- R₆ représente un atome d'hydrogène, un radical hydroxyle, amino ou alkyle en C₁-C₄ ;
- R₇ représente un atome d'hydrogène ou un radical alkyle en C₁-C₄ ;
- R₈ représente un atome d'hydrogène ou d'halogène tel que le chlore, le brome, l'iode ou le fluor, ou un radical amino ;
- R₉ représente un atome d'hydrogène, un radical hydroxyle, amino, alcoxy en C₁-C₄, monohydroxyalcoxy en C₁-C₄ ou polyhydroxyalcoxy en C₂-C₄ ;
au moins deux des radicaux R₅ à R₉ étant différents d'un atome d'hydrogène ; et ladite composition étant exempte de tout système enzymatique susceptible de provoquer l'oxydation des composés de formule (I) et/ou (II).
Comme indiqué précédemment, la composition tinctoriale conforme à l'invention conduit à des colorations puissantes qui présentent de plus d'excellentes propriétés de résistance vis à vis de l'action des différents agents extérieurs (lumière, intempéries, lavage, ondulation permanente, transpiration, frottements).

L'invention a également pour objet un procédé de teinture d'oxydation des fibres kératiniques mettant en oeuvre cette composition tinctoriale.

Les pyrazolo-[1,5-a]-pyrimidines de formule (I) utilisables à titre de base d'oxydation dans la composition tinctoriale conforme à l'invention sont des composés connus et décrits dans la demande de brevet FR-A-2 750 048.

Parmi les pyrazolo-[1,5-a]-pyrimidines de formule (I), utilisables à titre de base d'oxydation dans les compositions tinctoriales conformes à l'invention, on peut notamment citer :
- la pyrazolo-[1,5-a]-pyrimidine-3,7-diamine ;
- la 2-méthyl pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol ;
- le 3-amino 5-méthyl pyrazolo-[1,5-a]-pyrimidin-7-ol ;
- le 3-amino pyrazolo-[1,5-a]-pyrimidin-5-ol ;
- le 2-(3-amino pyrazolo-[1,5-a]-pyrimidin-7-ylamino)-éthanol ;
- la 3-amino-7-β-hydroxyéthylamino-5-méthyl-pyrazolo-[1,5-a]-pyrimidine;
- le 2-(7-amino pyrazolo-[1,5-a]-pyrimidin-3-ylamino)-éthanol ;
- le 2-[(3-amino-pyrazolo-[1,5-a]-pyrimidin-7-yl)-(2-hydroxyéthyl)-amino]-éthanol ;
- le 2-[(7-amino-pyrazolo-[1,5-a]-pyrimidin-3-yl)-(2-hydroxyéthyl)-amino]-éthanol ;
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N-7, N-7-tetraméthyl pyrazolo-[1,5-a]-pyrimidine-3,7-diamine ;
et leurs sels d'addition avec un acide ou avec une base.

Parmi les pyridines de formule (II), utilisables à titre de coupleur dans les compositions tinctoriales conformes à l'invention, on peut notamment citer :
- la 2,6-dihydroxy 3,4-diméthyl pyridine,
- la 5-chloro 2,3-dihydroxy pyridine,
- la 3,5-diamino 2,6-diméthoxy pyridine,
- la 3-amino 2-(β-hydroxyéthyl)amino 6-méthoxy pyridine,
- la 2,6-bis-(β-hydroxyéthyloxy) 3,5-diamino pyridine,
- la 3-amino 5-hydroxy 2,6-diméthoxy pyridine,
- la 3-amino 2-méthylamino 6-méthoxy pyridine,
- la 2-amino 3-hydroxy pyridine,
- la 2-diméthylamino 5-amino pyridine,
- la 2,6-diamino pyridine,
- la 3,5-diamino 2-(β,γ-dihydroxy)propyloxy pyridine,
- la 3,5-diamino 2-(γ-hydroxypropyloxyéthyloxy) pyridine,
et leurs sels d'addition avec un acide.

D'une manière générale, les sels d'addition avec un acide utilisables dans le cadre de l'invention (bases d'oxydation et coupleurs) sont notamment choisis parmi les chlorhydrates, les bromhydrates, les sulfates, les citrates, les succinates, les tartrates, les lactates et les acétates. Les sels d'addition avec une base utilisables dans le cadre des compositions tinctoriales de l'invention sont notamment ceux obtenus avec la soude, la potasse, l'ammoniaque ou les amines.

La ou les pyrazolo-[1,5-a]-pyrimidines de formule (I) conformes à l'invention et/ou le ou leurs sels d'addition avec un acide ou avec une base représentent de préférence de 0,0005 à 12 % en poids environ du poids total de la composition tinctoriale, et encore plus préférentiellement de 0,005 à 6 % en poids environ de ce poids.

La ou les pyridines de formule (II) conformes à l'invention et/ou le ou leurs sels d'addition avec un acide représentent de préférence de 0,0001 à 10 % en poids environ du poids total de la composition tinctoriale et encore plus préférentiellement de 0,005 à 5 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (III) suivante : 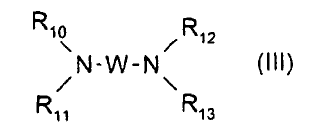 dans laquelle W est un reste propylène éventuellement substitué par un groupement hydroxyle ou un radical alkyle en C₁-C₆ ; R₁₀, R₁₁, R₁₂ et R₁₃, identiques ou différents, représentent un atome d'hydrogène, un radical alkyle en C₁-C₆ ou hydroxyalkyle en C₁-C₆.

La composition tinctoriale conforme à l'invention peut également renfermer au moins un colorant direct, notamment pour modifier les nuances ou les enrichir en reflets.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques tels que par exemple des gommes de guar non-ioniques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de poudres, de crèmes, de gels, ou sous toute autre forme appropriée pour réaliser une teinture des fibres kératiniques, et notamment des cheveux humains.

L'invention a également pour objet un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant non enzymatique qui est ajouté juste au moment de l'emploi à la composition tinctoriale ou qui est présent dans une composition oxydante appliquée simultanément ou séquentielle ment.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant non enzymatique présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les peracides et les persels tels que les perborates et persulfates,. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples suivants sont destinés à illustrer l'invention sans pour autant présenter un caractère limitatif.

### EXEMPLES

### EXEMPLES 1 à 3 DE TEINTURE EN MILIEU ALCALIN

On a préparé les compositions tinctoriales conformes à l'invention suivantes, (teneurs en grammes) :

| **EXEMPLE** | **1** | **2** | **3** |
|---|---|---|---|
| 3,7-diaminopyrazolopyrimidine, 2HCl (base d'oxydation de formule (I)) | 0,666 | 0,666 | 0,666 |
| 2-amino 3-hydroxy pyridine (coupleur de formule (II)) | 0,333 | - | - |
| 2,6-diamino pyridine (coupleur de formule (II)) | - | 0,333 | - |
| 3,5-diamino 2-(γ-hydroxypropyloxyéthyloxy) pyridine, 2 HCl (coupleur de formule (II)) | - | - | 0,858 |
| Support de teinture commun n°1 | (*) | (*) | (*) |
| Eau déminéralisée q.s.p. | 100 g | 100 g | 100 g |

### (*) Support de teinture commun n°1 :

- Alcool éthylique à 96° 18 g
- Métabisulfite de sodium en solution aqueuse à 35% 0,68 g
- Sel pentasodique de l'acide diéthylènetriaminopentacétique 1,1 g
- Ammoniaque à 20% de NH₃ 10,0 g

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris naturels à 90 % de blancs pendant 30 minutes. Les mèches ont ensuite été rincés, lavés avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| **1** | 10 ± 0,2 | Rouge cendré irisé |
| **2** | 10 ± 0,2 | Rouge violine |
| **3** | 10 *±* 0.2 | Rouge violine |

### EXEMPLES 4 à 6 DE TEINTURE EN MILIEU NEUTRE

On a préparé les compositions tinctoriales conformes à l'invention suivantes, (teneurs en grammes) :

| **EXEMPLE** | **4** | **5** | **6** |
|---|---|---|---|
| 3,7-diaminopyrazolopyrimidine, 2HCl (base d'oxydation de formule (I)) | 0,666 | 0,666 | 0,666 |
| 2,6-dihydroxy 4-méthyl pyridine (coupleur de formule (II)) | 0,345 | - | - |
| 2,6-dihydroxy 3,4-diméthyl pyridine (coupleur de formule (II)) | - | 0,417 | - |
| 3,5-diamino 2-(β,γ-dihydroxy)propyloxy pyridine, 2HCl (coupleur de formule (II)) | - | - | 0,816 |
| Support de teinture commun n°2 | (**) | (**) | (**) |
| Eau déminéralisée qsp | 100 g | 100 g | 100 g |

### (**) Support de teinture commun n°2 :

- Ethanol à 96° 18 g
- Tampon K₂HPO₄/KH₂PO₄ (1,5 M / 1 M) 10 g
- Métabisulfite de sodium 0,68 g
- Sel pentasodique de l'acide diéthylènetriaminopentacétique 1,1 g

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris naturels à 90 % de blancs pendant 30 minutes. Les mèches ont ensuite été rincés, lavés avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| **4** | 5,7 ± 0,2 | Rouge cendré irisé |
| **5** | 5,7 ± 0,2 | Irisé |
| **6** | 5,7 ± 0,2 | Rouge violine |

## Revendications

1. Composition pour la teinture d'oxydation des fibres kératiniques, et en particulier des fibres kératiniques humaines telles que les cheveux, **caractérisée par le fait qu'**elle contient, dans un milieu approprié pour la teinture :
- au moins une base d'oxydation choisie parmi les pyrazolo-[1,5-a]-pyrimidines de formule (I) suivante, et leurs sels d'addition avec un acide ou avec une base : 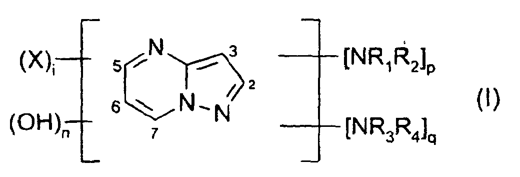 dans laquelle :
- R₁, R₂ R₃ et R₄ désignent, identiques ou différents un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical amino alkyle en C₁-C₄ (l'amine pouvant être protégée par un acétyle, un uréido, un sulfonyl), un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C₄)alkyl- ou di-[hydroxy(C₁-C₄) alkyl]-amino alkyle en C₁-C₄ ;
- les radicaux X, identiques ou différents, désignent un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C₄)alkyl ou di-[hydroxy(C₁-C₄)alkyl]amino alkyle en C₁-C₄, un radical amino, un radical (C₁-C₄)alkyl- ou di-[(C₁-C₄)alkyl]-amino ; un atome d'halogène, un groupe acide carboxylique, un groupe acide sulfonique ;
- i vaut 0, 1, 2 ou 3;
- p vaut 0 ou 1 ;
- q vaut 0 ou 1 ;
- n vaut 0 ou 1 ;
sous réserve que :
- (i) la somme p + q est différente de 0 ;
- (ii) lorsque p + q est égal à 2, alors n vaut 0 et les groupes NR₁R₂ et NR₃R₄ occupent les positions (2,3) ; (5,6) ; (6,7) ; (3,5) ou (3,7) ;
- (iii) lorsque p + q est égal à 1 alors n vaut 1 et le groupe NR₁R₂ (ou NR₃R₄) et le groupe OH occupent les positions (2,3) ; (5,6) ; (6,7) ; (3,5) ou (3,7) ;
- et au moins un coupleur choisi parmi les pyridines de formule (II) suivante, et leurs sels d'addition avec un acide : 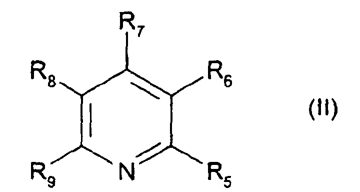 dans laquelle :
- R₅ représente un atome d'hydrogène, un radical hydroxyle, amino, alcoxy en C₁-C₄, mono- ou di-alky(C₁-C₄)amino, monohydroxyalkyl(C₁-C₄)amino, polyhydroxyalkyl(C₂-C₄)amino, monohydroxyalcoxy en C₁-C₄, polyhydroxyalcoxy en C₂-C₄, alcoxy en C₁-C₄, ou monohydroxyalcoxy(C₁-C₄)alcoxy en C₁-C₄ ;
- R₆ représente un atome d'hydrogène, un radical hydroxyle, amino ou alkyle en C₁-C₄ ;
- R₇ représente un atome d'hydrogène ou un radical alkyle en C₁-C₄ ;
- R₈ représente un atome d'hydrogène ou d'halogène tel que le chlore, le brome, l'iode ou le fluor, ou un radical amino ;
- R₉ représente un atome d'hydrogène, un radical hydroxyle, amino, alcoxy en C₁-C₄, monohydroxyalcoxy en C₁-C₄ ou polyhydroxyalcoxy en C₂-C₄ ; au moins deux des radicaux R₅ à R₉ étant différents d'un atome d'hydrogène ; et ladite composition étant exempte de tout système enzymatique susceptible de provoquer l'oxydation des composés de formule (I) et/ou (II).

2. Composition selon la revendication 1, **caractérisée par le fait que** les pyrazolo-[1,5-a]-pyrimidines de formule (I) sont choisies parmi :
- la pyrazolo-[1,5-a]-pyrimidine-3,7-diamine ;
- la 2-méthyl pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol ;
- le 3-amino 5-méthyl pyrazolo-[1,5-a]-pyrimidin-7-ol ;
- le 3-amino pyrazolo-[1,5-a]-pyrimidin-5-ol ;
- le 2-(3-amino pyrazolo-[1,5-a]-pyrimidin-7-ylamino)-éthanol ;
- la 3-amino-7-β-hydroxyéthylamino-5-méthyl-pyrazolo-[1,5-a]-pyrimidine;
- le 2-(7-amino pyrazolo-[1,5-a]-pyrimidin-3-ylamino)-éthanol ;
- le 2-[(3-amino-pyrazolo-[1,5-a]-pyrimidin-7-yl)-(2-hydroxyéthyl)-amino]-éthanol ;
- le 2-[(7-amino-pyrazolo-[1,5-a]-pyrimidin-3-yl)-(2-hydroxyéthyl)-amino]-éthanol ;
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N-7, N-7-tetraméthyl pyrazolo-[1,5-a]-pyrimidine-3,7-diamine ;
et leurs sels d'addition avec un acide ou avec une base.

3. Composition selon la revendication 1 ou 2, **caractérisée par le fait que** les pyridines de formule (II) sont choisies parmi :
- la 2,6-dihydroxy 3,4-diméthyl pyridine,
- la 5-chloro 2,3-dihydroxy pyridine,
- la 3,5-diamino 2,6-diméthoxy pyridine,
- la 3-amino 2-(β-hydroxyéthyl)amino 6-méthoxy pyridine,
- la 2,6-bis-(β-hydroxyéthyloxy) 3,5-diamino pyridine,
- la 3-amino 5-hydroxy 2,6-diméthoxy pyridine,
- la 3-amino 2-méthylamino 6-méthoxy pyridine,
- la 2-amino 3-hydroxy pyridine,
- la 2-diméthylamino 5-amino pyridine,
- la 2,6-diamino pyridine,
- la 3,5-diamino 2-(β,γ-dihydroxy)propyloxy pyridine,
- la 3,5-diamino 2-(γ-hydroxypropyloxyéthyloxy) pyridine,
et leurs sels d'addition avec un acide.

4. Composition selon l'une quelconque des revendications 1 à 3, **caractérisée par le fait que** les sels d'addition avec un acide sont choisis parmi les chlorhydrates, les bromhydrates, les sulfates, les citrates, les succinates, les tartrates, les lactates et les acétates et que les sels d'addition avec une base sont choisis parmi ceux obtenus avec la soude, la potasse, l'ammoniaque ou les amines.

5. Composition selon l'une quelconque des revendications précédentes, **caractérisée par le fait que** la ou les pyrazolo-[1,5-a]-pyrimidines de formule (I) et/ou le ou leurs sels d'addition avec un acide ou avec une base représentent de 0,0005 à 12% en poids du poids total de la composition tinctoriale.

6. Composition la revendication 5, **caractérisée par le fait que** la ou les pyrazolo-[1,5-a]-pyrimidines de formule (I) et/ou le ou leurs sels d'addition avec un acide ou avec une base représentent de 0,005 à 6% en poids du poids total de la composition tinctoriale.

7. Composition selon l'une quelconque des revendications précédentes, **caractérisée par le fait que** la ou les pyridines de formule (II) et/ou le ou leurs sels d'addition avec un acide représentent de 0,0001 à 10 % en poids du poids total de la composition tinctoriale.

8. Composition selon la revendication 7, **caractérisée par le fait que** la ou les pyridines de formule (II) et/ou le ou leurs sels d'addition avec un acide représentent de 0,005 à 5 % en poids du poids total de la composition tinctoriale.

9. Procédé de teinture d'oxydation des fibres kératiniques et en particulier des fibres kératiniques humaines telles que les cheveux **caractérisé par le fait qu'**on applique sur lesdites fibres au moins une composition tinctoriale telle que définie à l'une quelconque des revendications 1 à 8, et que l'on révèle la couleur à pH acide, neutre ou alcalin à l'aide d'un agent oxydant non enzymatique qui est ajouté juste au moment de l'emploi à la composition tinctoriale ou qui est présent dans une composition oxydante appliquée simultanément ou séquentiellement.

10. Procédé selon la revendication 9, **caractérisé par le fait que** l'agent oxydant est choisi parmi le peroxyde d'hydrogène, le peroxyde d'urée, les bromates de métaux alcalins, les peracides et les persels.

11. Dispositif à plusieurs compartiments, ou "kit" de teinture à plusieurs compartiments, dont un premier compartiment renferme une composition tinctoriale telle que définie à l'une quelconque des revendications 1 à 8 et un second compartiment renferme une composition oxydante contenant un agent oxydant non enzymatique.

## Claims

1. Composition for the oxidation dyeing of keratinous fibres and in particular of human keratinous fibres, such as hair, **characterized in that** it comprises, in a medium appropriate for dyeing:
- at least one oxidation base chosen from pyrazolo[1,5-a]pyrimidines of following formula (I) and their addition salts with an acid or with a base: 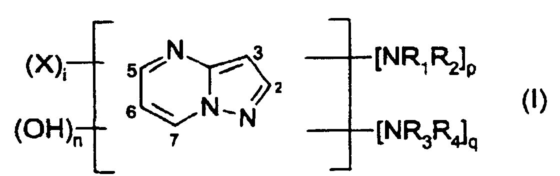 in which:
- R₁, R₂, R₃ and R₄, which are identical or different, denote a hydrogen atom, a (C₁-C₄)alkyl radical , an aryl radical, a hydroxy(C₁-C₄)alkyl radical, a polyhydroxy (C₂-C₄) alkyl radical, a (C₁-C₄)alkoxy(C₁-C₄)-alkyl radical, an amino(C₁-C₄)alkyl radical (it being possible for the amine to be protected by an acetyl, a ureido or a sulphonyl), a (C₁-C₄)alkylamino(C₁-C₄)alkyl radical, a di[(C₁-C₄)alkyl]amino(C₁-C₄)alkyl radical (it being possible for the dialkyls to form a 5- or 6-membered aliphatic or heterocyclic ring), a hydroxy(C₁-C₄)alkylamino(C₁-C₄)alkyl radical or a di [hydroxy(C₁-C₄)alkyl]amino(C₁-C₄)alkyl radical;
- the X radicals, which are identical or different, denote a hydrogen atom, a (C₁-C₄)alkyl radical, an aryl radical, a hydroxy(C₁-C₄)alkyl radical, a polyhydroxy(C₂-C₄)alkyl radical, an amino(C₁-C₄)alkyl radical, a (C₁-C₄)alkylamino(C₁-C₄)alkyl radical, a di[(C₁-C₄)alkyl]amino(C₁-C₄)alkyl radical (it being possible for the dialkyls to form a 5- or 6-membered aliphatic or heterocyclic ring), a hydroxy (C₁-C₄)alkylamino(C₁-C₄)alkyl radical , a di[hydroxy(C₁-C₄)alkyl]amino(C₁-C₄)alkyl radical, an amino radical, a (C₁-C₄)alkylamino radical, a di[(C₁-C₄)alkyl] amino radical, a halogen atom, a carboxylic acid group or a sulphonic acid group;
- i has the value 0, 1, 2 or 3;
- p has the value 0 or 1;
- q has the value 0 or 1;
- n has the value 0 or 1;
with the proviso that:
- (i) the sum p + q is other than 0;
- (ii) when p + q is equal to 2, then n has the value 0 and the NR₁R₂ and NR₃R₄ groups occupy the (2,3), (5,6), (6,7), (3,5) or (3,7) positions;
- (iii) when p + q is equal to 1, then n has the value 1 and the NR₁R₂ (or NR₃R₄) group and the OH group occupy the (2,3), (5,6), (6,7), (3,5) or (3,7) positions;
- and at least one coupler chosen from pyridines of following formula (II) and their addition salts with an acid: 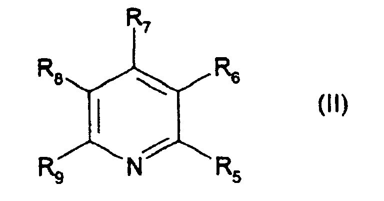 in which:
- R₅ represents a hydrogen atom or a hydroxyl, amino, (C₁-C₄)alkoxy, mono- or di(C₁-C₄)alkylamino, monohydroxy(C₁-C₄)alkylamino, polyhydroxy (C₂-C₄) alkylamino, monohydroxy(C₁-C₄)alkoxy, polyhydroxy(C₂-C₄)alkoxy or monohydroxy(C₁-C₄)alkoxy(C₁-C₄)alkoxy radical;
- R₆ represents a hydrogen atom or a hydroxyl, amino or (C₁-C₄)alkyl radical ;
- R₇ represents a hydrogen atom or a (C₁-C₄)alkyl radical;
- R₈ represents a hydrogen or halogen atom, such as chlorine, bromine, iodine or fluorine, or an amino radical;
- R₉ represents a hydrogen atom or a hydroxyl, amino, (C₁-C₄)alkoxy, monohydroxy (C₁-C₄) alkoxy or polyhydroxy(C₂-C₄)alkoxy radical;
at least two of the R₅ to R₉ radicals being other than a hydrogen atom, and the said composition being devoid of any enzymatic system capable of bringing about oxidation of the compounds of formula (I) and/or (II).

2. Composition according to Claim 1, **characterized in that** the pyrazolo[1,5-a]pyrimidines of formula (I) are chosen from:
- pyrazolo[1,5-a]pyrimidine-3,7-diamine;
- 2-methylpyrazolo[1,5-a]pyrimidine-3,7-diamine;
- 2,5-dimethylpyrazolo[1,5-a]pyrimidine-3,7-diamine;
- pyrazolo[1,5-a]pyrimidine-3,5-diamine;
- 2,7-dimethylpyrazolo[1,5-a]pyrimidine-3,5-diamine;
- 3-aminopyrazolo[1,5-a]pyrimidin-7-ol;
- 3-amino-5-methylpyrazolo[1,5-a]pyrimidin-7-ol;
- 3-aminopyrazolo[1,5-a]pyrimidin-5-ol;
- 2-(3-aminopyrazolo[1,5-a]pyrimidin-7-ylamino)ethanol;
- 3-amino-7-(β-hydroxyethylamino)-5-methylpyrazolo-[1,5-a]pyrimidine;
- 2-(7-aminopyrazolo[1,5-a]pyrimidin-3-ylamino)ethanol;
- 2-[(3-aminopyrazolo[1,5-a]pyrimidin-7-yl)(2-hydroxyethyl)amino]ethanol;
- 2-[(7-aminopyrazolo[2,5-a]pyrimidin-3-yl)(2-hydroxyethyl)amino]ethanol;
- 5,6-dimethylpyrazolo[1,5-a]pyrimidine-3,7-diamine;
- 2,6-dimethylpyrazolo[1,5-a]pyrimidine-3,7-diamine;
- 2,5,N-7,N-7-tetramethylpyrazolo[1,5-a]pyrimidine-3,7-diamine;
and their addition salts with an acid or with a base.

3. Composition according to Claim 1 or 2, **characterized in that** the pyridines of formula (II) are chosen from:
- 2,6-dihydroxy-3,4-dimethylpyridine,
- 5-chloro-2,3-dihydroxypyridine,
- 3,5-diamino-2,6-dimethoxypyridine,
- 3-amino-2-(β-hydroxyethyl)amino-6-methoxypyridine,
- 2,6-bis(β-hydroxyethyloxy)-3,5-diaminopyridine,
- 3-amino-5-hydroxy-2,6-dimethoxypyridine,
- 3-amino-2-methylamino-6-methoxypyridine,
- 2-amino-3-hydroxypyridine,
- 2-dimethylamino-5-aminopyridine,
- 2,6-diaminopyridine,
- 3,5-diamino-2-(β,γ-dihydroxypropyloxy)pyridine,
- 3,5-diamino-2-(γ-hydroxypropyloxyethyloxy)pyridine,
and their addition salts with an acid.

4. Composition according to any one of Claims 1 to 3, **characterized in that** the addition salts with an acid are chosen from hydrochlorides, hydrobromides, sulphates, citrates, succinates, tartrates, lactates and acetates and **in that** the addition salts with a base are chosen from those obtained with sodium hydroxide, potassium hydroxide, aqueous ammonia or amines.

5. Composition according to any one of the preceding claims, **characterized in that** the pyrazolo[1,5-a]pyrimidine or pyrazolo[1,5-a]pyrimidines of formula (I) and/or the addition salt or their addition salts with an acid or with a base represent from 0.0005 to 12% by weight of the total weight of the dyeing composition.

6. Composition according to Claim 5, **characterized in that** the pyrazolo[1,5-a]pyrimidine or pyrazolo[1,5-a]pyrimidines of formula (I) and/or the addition salt or their addition salts with an acid or with a base represent from 0.005 to 6% by weight of the total weight of the dyeing composition.

7. Composition according to any one of the preceding claims, **characterized in that** the pyridine or pyridines of formula (II) and/or the addition salt or their addition salts with an acid represent from 0.0001 to 10% by weight of the total weight of the dyeing composition.

8. Composition according to Claim 7, **characterized in that** the pyridine or pyridines of formula (II) and/or the addition salt or their addition salts with an acid represent from 0.005 to 5% by weight of the total weight of the dyeing composition.

9. Process for the oxidation dyeing of keratinous fibres and in particular human keratinous fibres, such as hair, **characterized in that** at least one dyeing composition as defined in any one of Claims 1 to 8 is applied to the said fibres and **in that** the colour is developed at acidic, neutral or alkaline pH using a non-enzymatic oxidizing agent which is added only at the time of use to the dyeing composition or which is present in an oxidizing composition applied simultaneously or sequentially.

10. Process according to Claim 9, **characterized in that** the oxidizing agent is chosen from hydrogen peroxide, urea hydrogen peroxide, alkali metal bromates, peracids and persalts.

11. Dyeing multi-compartment device or kit with several compartments, a first compartment of which includes a dyeing composition as defined in any one of Claims 1 to 8 and a second compartment of which includes an oxidizing composition comprising a non-enzymatic oxidizing agent.

## Patentansprüche

1. Zusammensetzung zum oxidativen Färben von Keratinfasern und insbesondere menschlichen Keratinfasern, wie dem Haar, **dadurch gekennzeichnet, dass** sie in einem zum Färben geeigneten Medium enthält:
- mindestens eine Oxidationsbase, die unter den Pyrazolo-[1,5-a]-pyrimidinen der folgenden Formel (I) und den Additionssalzen dieser Verbindungen mit einer Säure oder einer Base ausgewählt ist: 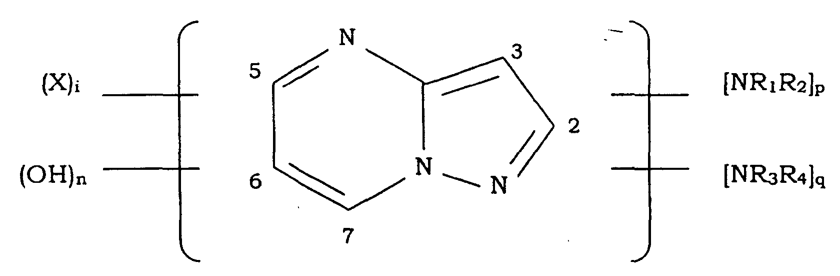 worin bedeuten:
- R₁, R₂, R₃ und R₄, die identisch oder voneinander verschieden sind, Wasserstoff, C₁₋₄-Alkyl, Aryl, C₁₋₄-Hydroxyalkyl, C₂₋₄-Polyhydroxyalkyl, C₁₋₄-Alkoxy-C₁₋₄-alkyl, C₁₋₄-Aminoalkyl (wobei die Aminogruppe mit Acetyl, Ureido oder Sulfonyl geschützt sein kann), C₁₋₄-Alkylamino-C₁₋₄-alkyl, Di-[C₁₋₄-alkyl]amino-C₁₋₄alkyl (wobei die beiden Alkylgruppen einen aliphatischen oder heterocyclischen Ring mit 5 oder 6 Gliedern bilden können), Hydroxy-C₁₋₄-alkylamino-C₁₋₄-alkyl oder Di-[hydroxy-C₁₋₄-alkyl]-amino-C₁₋₄-alkyl;
- die Gruppen X, die identisch oder voneinander verschieden sind, Wasserstoff, C₁₋₄-Alkyl, Aryl, C₁₋₄-Hydroxyalkyl, C₂₋₄-Polyhydroxyalkyl, C₁₋₄-Aminoalkyl, C₁₋₄-Alkylamino-C₁₋₄-alkyl, Di-[C₁₋₄-alkyl]amino-C₁₋₄-alkyl (wobei die beiden Alkylgruppen einen aliphatischen oder heterocyclischen Ring mit 5 oder 6 Gliedern bilden können), Hydroxy-C₁₋₄-alkylamino-C₁₋₄-alkyl, Di-[Hydroxy-C₁₋₄-alkyl]amino-C₁₋₄-alkyl, Amino, C₁₋₄-Alkylamino, Di-[C₁₋₄-alkyl]amino, Halogen, Carboxy oder eine Sulfonsäuregruppe;
- i 0, 1, 2 oder 3;
- p 0 oder 1:
- q 0 oder 1;
- n 0 oder 1;
mit der Maßgabe, dass
(i) die Summe p + q von Null verschieden ist;
(ü) n Null bedeutet und die Gruppen NR₁R₂ und NR₃R₄ die Stellungen (2,3); (5,6); (6,7); (3,5) oder (3,7) einnehmen, wenn p + q 2 ist;
(iii) n 1 bedeutet und die Gruppe NR₁R₂ (oder NR₃R₄) und die OH-Gruppe die Stellungen (2,3); (5,6); (6,7); (3,5) oder (3,7) einnehmen, wenn p + q 1 ist; und
- mindestens einen Kuppler, der unter den Pyridinen der folgenden Formel (II) und deren Additionssalzen mit einer Säure ausgewählt ist: 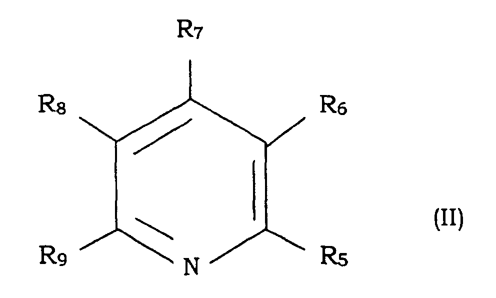 worin bedeuten:
- R₅ Wasserstoff, Hydroxy, Amino, C₁₋₄-Alkoxy, C₁₋₄-Monoalkylamino, C₁₋₄-Dialkylamino, Monohydroxy-C₁₋₄-alkylamino, Polyhydroxy-C₂₋₄-alkylamino, C₁₋₄-Monohydroxyalkoxy, C₂₋₄-Polyhydroxyalkoxy, C₁₋₄-Alkoxy, C₁₋₄-Monohydroxyalkoxy-C₁₋₄-alkoxy,
- R₆ Wasserstoff, Hydroxy, Amino oder C₁₋₄-Alkyl,
- R₇ Wasserstoff oder C₁₋₄-Alkyl,
- R₈ Wasserstoff oder Halogen, wie Chlor, Brom, Iod oder Fluor, oder Amino,
- R₉ Wasserstoff, Hydroxy, Amino, C₁₋₄-Alkoxy, C₁₋₄-Monohydroxyalkoxy oder C₂₋₄-Polyhydroxyalkoxy,
wobei mindestens zwei der Gruppen R₅ bis R₉ von Wasserstoff verschieden sind und
wobei die Zusammensetzung kein enzymatisches System enthält, das befähigt ist, die Verbindungen der Formel (I) und/oder (II) zu oxidieren.

2. Zusammensetzung nach Anspruch 1, **dadurch gekennzeichnet, dass** die Pyrazolo-[1,5-a]-pyrimidine der Formel (I) ausgewählt sind unter:
- Pyrazolo-[1,5-a]-pyrimidin-3,7-diamin,
- 2-Methyl-pyrazolo-[1,5-a]-pyrimidin-3,7-diamin,
- 2,5-Dimethyl-pyrazolo-[1,5-a]-pyrimidin-3,7-diamin,
- Pyrazolo-[1,5-a]-pyrimidin-3,5-diamin,
- 2,7-Dimethyl-pyrazolo-[1,5-a]-pyrimidin-3,5-diamin,
- 3-Amino-pyrazolo-[1,5-a]-pyrimidin-7-ol,
- 3-Amino-5-methyl-pyrazolo-[1,5-a]-pyrimidin-7-ol,
- 3-Amino-pyrazolo-[1,5-a]-pyrimidin-5-ol,
- 2-(3-Amino-pyrazolo-[1,5-a]-pyrimidin-7-ylamino)-ethanol,
- 3-Amino-7-β-hydroxyethylamino-5-methyl-pyrazolo-[1,5-a]-pyrimidin,
- 2-(7-Amino-pyrazolo-[1,5-a]-pyrimidin-3-ylamino)-ethanol,
- 2-[(3-Amino-pyrazolo-[1,5-a]-pyrimidin-7-yl)-(2-hydroxyethyl)amino]-ethanol,
- 2-[(7-Amino-pyrazolo-[1,5-a]-pyrimidin-3-yl)-(2-hydroxyethyl)amino]-ethanol,
- 5,6-Dimethyl-pyrazolo-[1,5-a]-pyrimidin-3,7-diamin,
- 2,6-Dimethyl-pyrazolo-[1,5-a]-pyrimidin-3,7-diamin,
- 2,5,N7,N7-Tetramethyl-pyrazolo-[1,5-a]-pyrimidin-3,7-diamin
- und deren Additionssalzen mit einer Säure oder einer Base.

3. Zusammensetzung Anspruch 1 oder 2, **dadurch gekennzeichnet, dass** die Pyridine der Formel (II) ausgewählt sind unter:
- 2,6-Dihydroxy-3,4-dimethyl-pyridin,
- 5-Chlor-2,3-dihydroxy-pyridin,
- 3,5-Diamino-2,6-dimethoxy-pyridin,
- 3-Amino-2-(β-hydroxyethyl)amino-6-methoxy-pyridin,
- 2,6-Bis(β-hydroxyethyloxy)-3,5-diamino-pyridin,
- 3-Amino-5-hydroxy-2,6-dimethoxy-pyridin,
- 3-Amino-2-methylamino-6-methoxy-pyridin,
- 2-Amino-3-hydroxy-pyridin,
- 2-Dimethylamino-5-amino-pyridin,
- 2,6-Diamino-pyridin,
- 3,5-Diamino-2-(β,γ-dihydroxy)propyloxy-pyridin,
- 3,5-Diamino-2-(γ-hydroxypropyloxyethyloxy)-pyridin,
- und deren Additionssalzen mit einer Säure.

4. Zusammensetzung nach einem der Ansprüche 1 bis 3, **dadurch gekennzeichnet, dass** die Additionssalze mit einer Säure unter den Hydrochloriden, Hydrobromiden, Sulfaten, Citraten, Succinaten, Tartraten, Lactaten und Acetaten und die Additionssalze mit einer Base unter den Salzen ausgewählt sind, die mit Natriumhydroxid, Kaliumhydroxid, Ammoniak oder Aminen erhalten werden.

5. Zusammensetzung nach einem der vorhergehenden Ansprüche, **dadurch gekennzeichnet, dass** das oder die Pyrazolo-[1,5-a]-pyrimidine der Formel (I) und/ oder ihr(e) Additionssalz(e) mit einer Säure oder Base 0,0005 bis 12 Gew.-% des Gesamtgewichts der Farbmittelzusammensetzung ausmachen.

6. Zusammensetzung nach Anspruch 5, **dadurch gekennzeichnet, dass** das oder die Pyrazolo-[1,5-a]-pyrimidine der Formel (I) und/ oder ihr(e) Additionssalz(e) mit einer Säure oder Base 0,005 bis 6 Gew.-% des Gesamtgewichts der Farbmittelzusammensetzung ausmachen.

7. Zusammensetzung nach einem der vorhergehenden Ansprüche, **dadurch gekennzeichnet, dass** das oder die Pyridine der Formel (II) und/oder ihr(e) Additionssalz(e) mit einer Säure 0,0001 bis 10 Gew.-% des Gesamtgewichts der Farbmittelzusammensetzung ausmachen.

8. Zusammensetzung nach Anspruch 7, **dadurch gekennzeichnet, dass** das oder die Pyridine der Formel (II) und/ oder ihr(e) Additionssalz(e) mit einer Säure 0,005 bis 5 Gew.-% des Gesamtgewichts der Farbmittelzusammensetzung ausmachen.

9. Verfahren zum Färben von Keratinfasern und insbesondere menschlichen Keratinfasern, wie dem Haar, **dadurch gekennzeichnet, dass** auf die Fasern mindestens eine Farbmittelzusammensetzung nach einem der Ansprüche 1 bis 8 aufgebracht wird und die Farbe bei einem sauren, neutralen oder alkalischen pH-Wert mit einem nicht enzymatischen Oxidationsmittel entwickelt wird, das bei der Anwendung zu der Farbmittelzusammensetzung gegeben wird oder das in einer oxidierenden Zusammensetzung vorliegt, die gleichzeitig oder getrennt davon anschließend aufgetragen wird.

10. Verfahren nach Anspruch 9, **dadurch gekennzeichnet, dass** das Oxidationsmittel unter Wasserstoffperoxid, Harnstoffperoxid, Alkalimetallbromaten, Persäuren und Salzen von Persäuren ausgewählt ist.

11. Vorrichtung mit mehreren Abteilungen oder "Kit" zum Färben mit mehreren Abteilungen, wobei eine erste Abteilung eine Farbmittelzusammensetzung nach einem der Ansprüche 1 bis 8 und eine zweite Abteilung eine oxidierende Zusammensetzung mit einem nicht enzymatischen Oxidationsmittel enthält.
